# EUROPEAN PATENT SPECIFICATION

(11) **EP 2 745 790 B1**
(45) Date of publication and mention of the grant of the patent: **24.05.2017**
(21) Application number: 13198278.7
(22) Date of filing: 19.12.2013
(51) Int. Cl.: A61B 17/68, A61B 17/86, A61B 17/72

(54) **Percutaneous expanding hammertoe implant**
Perkutanes erweiterndes Hammerzehenimplantat
Implant pour orteil en marteau à extension percutanée

(30) Priority: 21.12.2012 US 201261745030 P; 29.03.2013 US 201313853399
(43) Date of publication of application: 25.06.2014
(73) Proprietor: Wright Medical Technology, Inc., Memphis, TN 38117 (US)
(72) Inventor: McCormick, Daniel, Germantown, Tennessee 38138 (US)
(74) Representative: Gerbino, Angelo

(56) References cited:
- WO-A1-01/80751
- WO-A2-02/34107
- WO-A2-2009/155577
- FR-A1- 2 728 779
- US-A- 4 237 875
- US-A- 5 470 230
- US-A1- 2005 177 158
- US-A1- 2011 066 190

## Description

### FIELD OF DISCLOSURE

The disclosed device generally relates to hammertoe correction implants and devices.

### BACKGROUND

A hammertoe or contracted toe is a deformity of the proximal inter-phalangeal joint of the second, third, or fourth toe causing it to be permanently bent and giving it a semblance of a hammer. Initially, hammertoes are flexible and may be corrected with simple measures but, if left untreated, hammertoes may require surgical intervention for correction. Persons with hammertoe may also have corns or calluses on the top of the middle joint of the toe or on the tip of the toe and may feel pain in their toes or feet while having difficulty finding comfortable shoes.

Various treatment strategies are available for correcting hammertoes. Conventionally, the first line of treatment for hammertoes includes employing new shoes having soft and spacious toe boxes. Additionally, toe exercises may be prescribed to stretch and strengthen respective muscles, e.g., gently stretching one's toes manually, using the toes to pick up things off the floor, etc. Another line of treatment may include employing straps, cushions or non-medicated corn pads to relieve symptoms. An addition method of treatment may include correction by surgery if other non-invasive treatment options fail. Conventional surgery usually involves inserting screws, wires or other similar implants in toes to straighten them. Traditional surgical methods generally include the use of Kirschner wires (K-wires). Due to various disadvantages of using K-wires, however, compression screws are being employed as a better implant alternative as K-wires require pings protruding through the end of respective toes due to their temporary nature. As a result, K-wires often lead to pin tract infections, loss of fixation, and other conditions. Additional disadvantages of K-wires include migration and breakage of the K-wires thus resulting in multiple surgeries.

Screw implants, however, may provide a more permanent solution as such implants do not need removal and thus have no protruding ends. Further, with the use of screw implants, a patient may wear normal footwear shortly after the respective surgery. Conventional screw implants possess a completely threaded body and do not provide a flexibility to the respective toe in its movement, i.e., conventional implants provide a pistoning effect. Furthermore, conventional screw implants are made for strong bones and are unsuitable for treatment of patients having weak bones which is a predominant reason why K-wire surgical implants are still employed despite their several disadvantages. Accordingly, there remains a need for developing hammertoe implants and devices which allow percutaneous implantation and can be expanded within the bone enabling enhanced fixation, faster operating and healing time.

### BRIEF DESCRIPTION OF THE DRAWINGS

Other objects, features, and advantages of the present subject matter will be apparent from the following description when read with reference to the accompanying drawings. In the drawings, wherein like reference numerals denote corresponding parts throughout the several views.
Figures 1A-1C illustrate views of an exemplary implant according to some embodiments of the present subject matter.
Figures 2A-2D illustrate views of another exemplary implant according to embodiments of the present subject matter.
Figure 3 illustrates an insertion method of an exemplary implant.
Figures 4A-4C illustrate views of a further exemplary implant.
Figure 5 illustrates an insertion method of an exemplary implant.

### DETAILED DESCRIPTION

With reference to the figures, where like elements have been given like numerical designations to facilitate an understanding of the present subject matter, the various embodiments of a percutaneous expanding hammertoe implant are described.

It should be noted that the figures are not necessarily to scale and certain features may be shown exaggerated in scale or in somewhat schematic form in the interest of clarity and conciseness. In the description, relative terms such as "horizontal," "vertical," "up," "down," "top" and "bottom" as well as derivatives thereof (e.g., "horizontally," "downwardly," "upwardly," etc.) should be construed to refer to the orientation as then described or as shown in the drawing figure under discussion. These relative terms are for convenience of description and normally are not intended to require a particular orientation. Terms including "inwardly" versus "outwardly," "longitudinal" versus "lateral" and the like are to be interpreted relative to one another or relative to an axis of elongation, or an axis or center of rotation, as appropriate. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise. When only a single machine is illustrated, the term "machine" shall also be taken to include any collection of machines that individually or jointly execute a set (or multiple sets) of instructions to perform any one or more of the methodologies discussed herein. The term "operatively connected" is such an attachment, coupling or connection that allows the pertinent structures to operate as intended by virtue of that relationship. In the claims, means-plus-function clauses, if used, are intended to cover the structures described, suggested, or rendered obvious by the written description or drawings for performing the recited function, including not only structural equivalents but also equivalent structures. The terms "implant" and "device" are used interchangeably in this disclosure and such use should not limit the scope of the claims appended herewith.

One embodiment of the present subject matter as specified in claim 1 provides a hammertoe implant or device having an inner and outer body positioned coaxially to each other. The outer body possesses axial slotted features and a threaded portion compatible with the threading of the inner body. Once the respective device is inserted to a desired depth and location, the inner body may be rotated while the outer body remains fixed in place. Rotation of the inner body may then cause the outer body to ride up axially due to the threaded engagement thereof. Thus, slotted features of the device may be forced to expand radially outward while simultaneously compressing the respective joint and anchoring the implant or device.

With reference to the figures herein, examples are given for percutaneous implantation for hammertoe corrective devices or implants. Exemplary implants can be driven into a predetermined location and expanded within the respective bone. This implantation may be accomplished with a single incision thus enabling a faster operating and healing time for the respective patient.

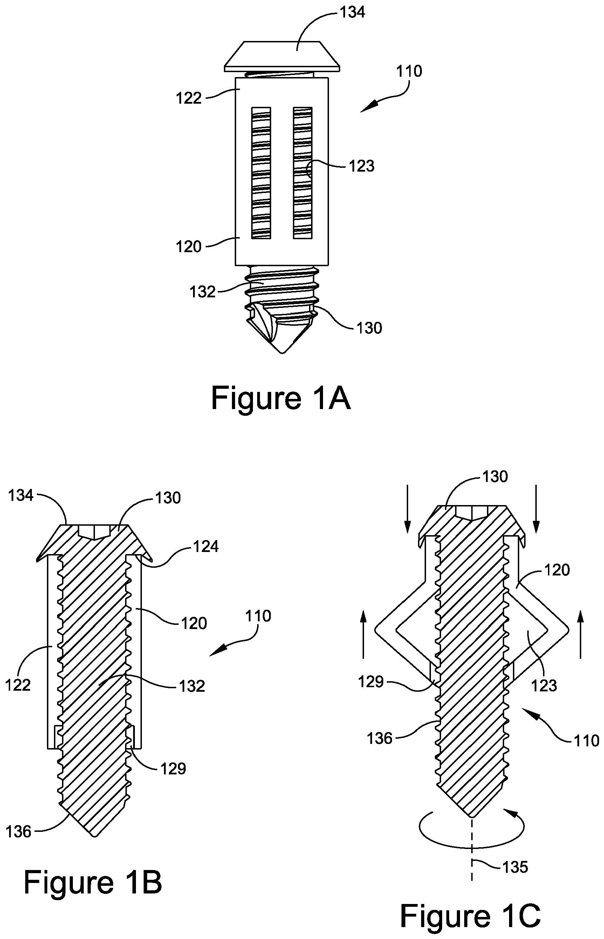

Figures 1A-1C illustrate views of an exemplary implant according to some embodiments of the present subject matter. Figure 1A is a front view of an exemplary hammertoe implant or device 110, Figure 1B is a cross section of the device of Figure 1A, and Figure 1C is a cross section of the device of Figure 1A in an engaged position. With reference to Figures 1A-1C, an exemplary hammertoe implant or device 110 includes an outer body 120 or sleeve and an inner body 130 or screw. The outer body 120, in some embodiments, comprises an annular sleeve 122 adaptable to accept the inner body 130. Generally, the outer diameter of the sleeve 122 substantially corresponds to the diameter of a pre-drilled hole in the receiving bone; however, the annular sleeve 122 may include features 123 which allow expansion of the sleeve 122 when the inner body 130 is fully engaged with the outer body 120 to thereby anchor the device 110 in a bone and compress a respective joint. In some embodiments of the present subject matter, the features 123 are one or more slots extending down the length of the sleeve 122. Of course, any number and arrangement of slots may be included in embodiments of the present subject matter and the illustration of two longitudinal running slots in Figure 1A should not limit the scope of the claims appended herewith. For example, the slots may extend in a spiral manner about the length of the sleeve and plural sets of slots may be arranged about the length or width of the sleeve.

With continued reference to Figures 1A-1C, the inner body 130 generally comprises a screw 132 having a head 134 and a threaded body 136 extending in a perpendicular direction from the head 134. The annular sleeve 122 includes a first region 124 adaptable to accept and mate to the head 134 of the screw 132 when the screw 132 is inserted into the annular sleeve 122. The annular sleeve 122 also includes a threaded region 129 which mates with the threaded body 136 of the screw 132. Once the screw 132 is inserted into and mated with the annular sleeve 122, the screw 132 may be rotated about its axis 135 (i.e., an axis of a pre-drilled hole in a receiving bone). As a function of the mating of the threaded region 129 of the sleeve 122 with the threaded body 136 of the screw 132, the screw 132 will pull the outer surface of the sleeve 122 against the joint line (not shown) thus ensuring that the sleeve 122 acts as an anchoring mechanism in the respective bone or joint and the screw 132 will act to compress the respective bone or joint.

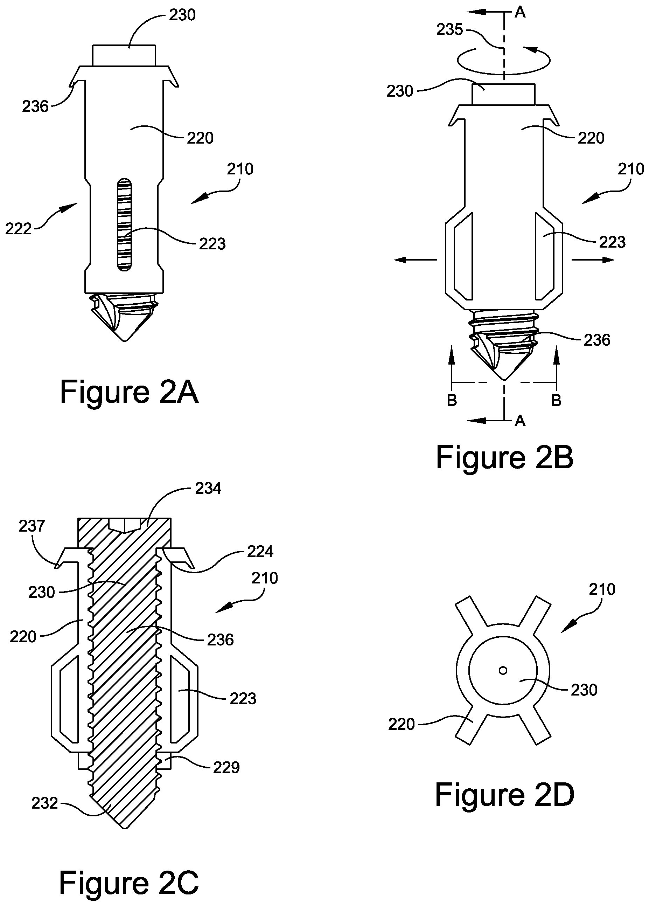

Figures 2A-2D illustrate views of another exemplary implant according to embodiments of the present subject matter. Figure 2A is a front view of an exemplary hammertoe implant or device 210, Figure 2B is a side view of the device of Figure 2A, Figure 2C is a cross section of the device of Figure 2B along line A-A, and Figure 2D is a cross section of the device of Figure 2B along line B-B. With reference to Figures 2A-2D, an exemplary hammertoe implant or device 210 includes an outer body 220 or sleeve and an inner body 230 or screw. The outer body 220, in some embodiments, comprises a substantially annular sleeve 222 adaptable to accept the inner body 230. Generally, the outer diameter of the sleeve 222 substantially corresponds to the diameter of a pre-drilled hole in the receiving bone; however, the annular sleeve 222 may include features 223 along a portion, or substantially all, of the sleeve which allow expansion of the sleeve 222 when the inner body 230 is fully engaged with the outer body 220 to anchor the device 210 in a bone and compress a respective joint. In some embodiments of the present subject matter, the features 223 may be one or more slots extending down a portion of the length of the sleeve 222. Of course, any number and arrangement of slots may be included in embodiments of the present subject matter and the illustration of two slots in Figures 2A-2C should not limit the scope of the claims appended herewith. For example, the slots may extend in a spiral manner about portions of the sleeve and plural sets of slots may be arranged about the sleeve. The sets may be arranged about the length or width of the sleeve. With continued reference to Figures 2A-2D, the inner body 230 generally comprises a screw 232 having a head 234 and a threaded body 236 extending in a perpendicular direction from the head 234. The annular sleeve 222 includes a first region 224 adaptable to accept and mate to the head 234 of the screw 232 when the screw 232 is inserted into the annular sleeve 222. The annular sleeve 222 also includes a hook feature 237 hidden during insertion of the implant 210 into the bone and exposed during expansion of the sleeve 222 to assist in an interface and/or anchor with the bone. The annular sleeve 222 also includes a threaded region 229 which mates with the threaded body 236 of the screw 232. Once the screw 232 is inserted into and mated with the annular sleeve 222, the screw 232 may be rotated about its axis 235 (i.e., an axis of a pre-drilled hole in a receiving bone). Hence one or more portions of the device 210 act as an anchoring mechanism (e.g., the hook feature 237, etc.) and the expansion of the annular sleeve 222 acts to drive compression of the respective bones in the joint. Thus, through the engagement and mating of the inner and outer bodies of the device, the outer body is forced to travel axially within a pre-drilled hole and deflect radially outwards to anchor the implant and compress at the middle region thereof.

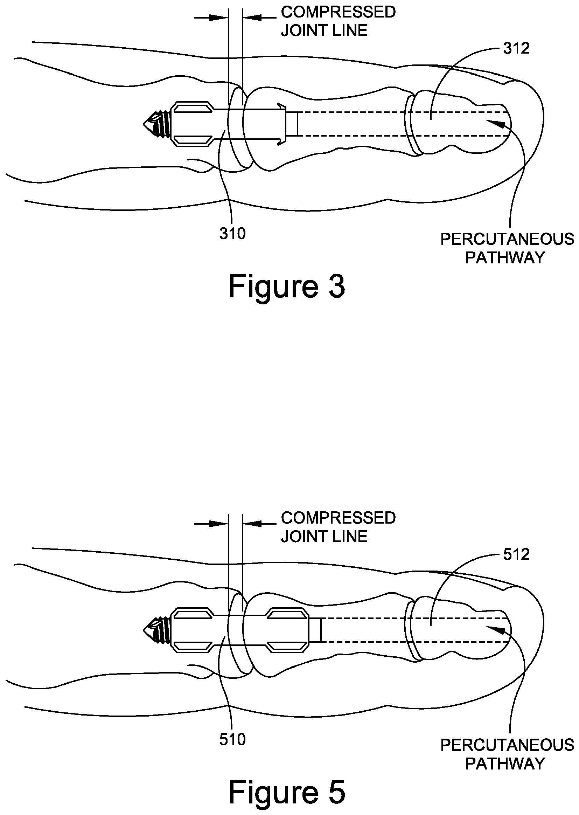

Figure 3 illustrates an insertion method. With reference to Figure 3, an exemplary implant 310 may be delivered to one or more of a patient's bones, in this case a metatarsal phalangeal joint, via a pre-drilled hole 312. Of course, the depicted metatarsal phalangeal joint should not limit the scope of the claims appended herewith as embodiments of the present subject matter may be employed in any number of bones and/or joints. Slotted features of the exemplary implant 310 as described above in relation to Figures 1A-2D will expand and anchor the distal end of the joint line. This may be achieved by mating threads of the inner and outer components or bodies the respective implant. For example, the head of the inner body may compress the opposing end of the joint line thus creating a secure construct.

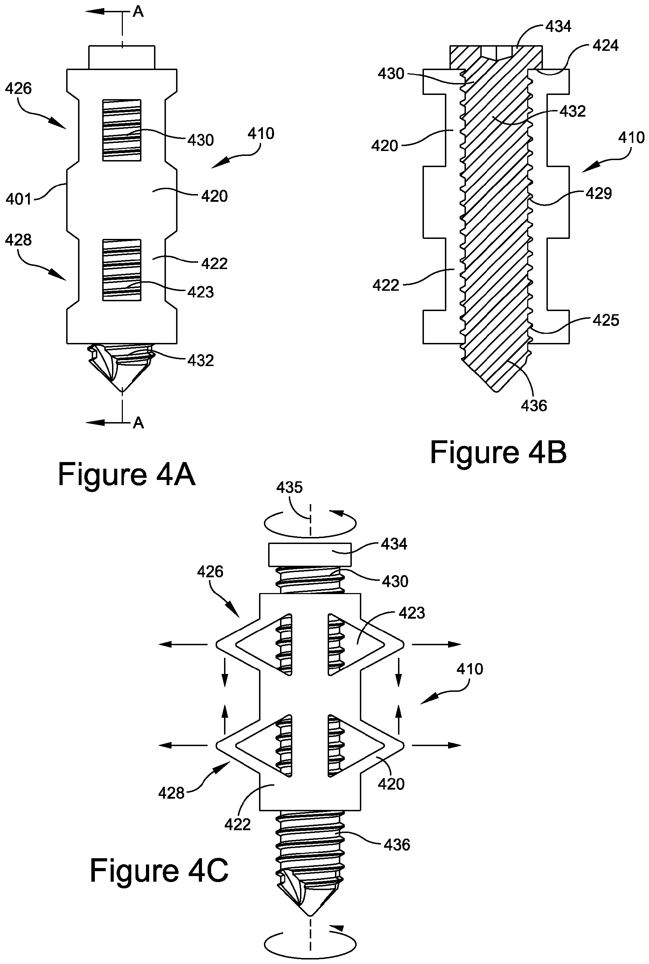

Figures 4A-4C illustrate views of a further exemplary implant. Figure 4A is a front view of an exemplary hammertoe implant or device 410, Figure 4B is a cross section of the device of Figure 4A along line A-A, Figure 4C is a side view of the device of Figure 4A in an engaged position. With reference to Figures 4A-4C, an exemplary hammertoe implant or device 410 includes an outer body 420 or sleeve and an inner body 430 or screw. The outer body 420, in some embodiments, comprises a substantially annular sleeve 422 adaptable to accept the inner body 430. Generally, the outer diameter 401 of the sleeve 422 substantially corresponds to the diameter of a pre-drilled hole in the receiving bone; however, the annular sleeve 422 may include features 423 along multiple portions of the sleeve which allow for expansion of the respective portions of the sleeve 422 when the inner body 430 is fully engaged with the outer body 420 to thereby anchor the device 410 in a bone and compress a respective joint. In some examples, the features 423 may be one or more slots extending down a first or upper portion 426 of the sleeve 422. The features 423 may also be included down a second or lower portion 428 of the sleeve 422. Of course, any number and arrangement of slots may be included in embodiments of the present subject matter and the illustration of two slots in both the upper and lower portions 426, 428 in Figures 4A-4C should not limit the scope of the claims appended herewith. For example, the slots may extend in a spiral manner about the respective portions of the sleeve and plural sets of slots may be arranged about the respective portions of the sleeve.

With continued reference to Figures 4A-4C, the inner body 430 generally comprises a screw 432 having a head 434 and a threaded body 436 extending in a perpendicular direction from the head 434. The annular sleeve 422 includes a first region 424 adaptable to accept and mate to the head 434 of the screw 432 when the screw 432 is inserted into the annular sleeve 422. The annular sleeve 422 may also include a first threaded region 429 which mates with a first section of the threaded body 436 of the screw 432 and a second threaded region 425 which mates with a second section of the threaded body 436 of the screw 432. In one embodiment, the first threaded region 429 has a right handed thread and the second threaded region 425 has a left handed thread. In another embodiment, the first threaded region 429 has a left handed thread and the second threaded region 425 has a right handed thread. Once the screw 432 is inserted into and mated with the annular sleeve 422, the screw 432 may be rotated about its axis 435 (i.e., an axis of a pre-drilled hole in a receiving bone). As a function of the mating of the threaded regions 424, 425 of the sleeve 422 with the threaded body 436 of the screw 432 and the rotation thereof, the screw 432 will pull portions of the outer surface of the sleeve 422 against the joint line (not shown) thus ensuring that one or more portions of the sleeve 422 (e.g., the slotted features) acts as an anchoring mechanism in the respective bone or joint will act to compress the respective bone or joint. Thus, through the engagement and mating of the inner and out bodies of the device, the outer body is forced to travel axially within a pre-drilled hole and deflect radially outwards to anchor the implant and compress at the middle region thereof.

Figure 5 illustrates an insertion method of an exemplary implant. With reference to Figure 5, an exemplary implant 510 may be delivered to one or more of a patient's bones, in this case a metatarsal phalangeal joint, via a pre-drilled hole 512. Slotted features of the exemplary implant 510 as described above in relation to Figures 4A-4C will expand and anchor the distal end of the joint line. This may be achieved by mating threads of the inner and outer components or bodies the respective implant. For example, the expanding outer body may compress the opposing end of the joint line thus creating a secure construct.

Although reference has been made to a patient's metatarsal phalangeal joint, one skilled in the art will understand that embodiments of the present subject matter may be implemented for other respective bones including, but not limited to other phalanges/digits and phalangeal/digital joints.

It may be emphasized that the above-described embodiments, particularly any "preferred" embodiments, are merely possible examples of implementations and merely set forth for a clear understanding of the principles of the disclosure. Many variations and modifications may be made to the above-described embodiments of the disclosure without departing substantially from the scope of the following claims.

As shown by the various configurations illustrated in Figures 1-5, a percutaneous expanding hammertoe implant have been described.

While preferred embodiments of the present subject matter have been described, it is to be understood that the embodiments described are illustrative only and that the scope of the invention is to be defined solely by the appended claims.

## Claims

1. A bone implant (110) comprising:
a screw (130) with a threaded end (136); and
an annular sleeve (122) accepting the screw (130), the sleeve (122) comprising:
a first threaded portion (129) to accept the threaded end (136) of the screw (130), and
an expanding feature (120) including at least two circumferentially arranged, closed end slots (123) separating at least two circumferentially arranged portions of said sleeve (122), said at least two slots (123) extending along the length of the sleeve (122) and said portions having an outer surface,
wherein the implant (110) is implanted into a joint through a percutaneous incision, and
wherein the screw (130) is rotated about its longitudinal axis when mated with the first threaded portion (129) of the sleeve (122) so as to radially expand the at least two circumferentially arranged portions of said sleeve (122) so that said outer surfaces expand and anchor the implant (110) in a respective bone,
said implant being **characterized in that** said annular sleeve (122) further comprises a hook (237) to anchor the implant in the respective bones.

2. The bone implant (110) of Claim 1 wherein said closed end slots (123) are selected from the group consisting of more slots arranged in a spiral down the length of the sleeve (122), a first set and a second set of slots each extending down disparate portions of the sleeve (122).

3. The bone implant (110) of Claim 1 wherein the bone for which the implant is suitable is a phalange selected from the group consisting of proximal phalange, intermediate phalange, distal phalange, and combinations thereof.

## Patentansprüche

1. Knochenimplantat (110), mit:
einer Schraube (130) mit einem mit einem Gewinde versehenen Ende (136); und
einer ringförmigen Hülse (122), die die Schraube (130) aufnimmt, wobei die Hülse (122) aufweist:
einen ersten mit einem Gewinde versehenen Bereich (129) zum Aufnehmen des mit einem Gewinde versehenen Endes (136) der Schraube (130), und
eine Erweiterungseinrichtung (120), die wenigstens zwei auf dem Umfang angeordnete, geschlossene Endschlitze (123) aufweist, die wenigstens zwei auf dem Umfang angeordnete Bereiche der Hülse (122) trennen, wobei die wenigstens zwei Schlitze (123) sich entlang der Länge der Hülse (122) erstrecken und die Bereiche eine Außenfläche aufweisen,
wobei das Implantat (110) durch einen perkutanen Schnitt in ein Gelenk eingesetzt wird, und
wobei die Schraube (130) sich um ihre Längsachse dreht, wenn sie in den ersten mit einem Gewinde versehenen Bereich (129) der Hülse (122) eingreift, so dass sie die wenigstens zwei auf dem Umfang angeordneten Bereiche der Hülse (122) radial aufweitet, so dass sich die Außenflächen aufweiten und das Implantat (110) in einem entsprechenden Knochen verankern,
wobei das Implantat **dadurch gekennzeichnet ist, dass** die ringförmige Hülse (122) ferner einen Haken (237) zum Verankern des Implantats in den entsprechenden Knochen aufweist.

2. Knochenimplantat (110) nach Anspruch 1, bei dem die geschlossenen Endschlitze (123) aus der Gruppe ausgewählt sind, die aus mehreren Schlitzen, die in einer Spirale abwärts der Länge der Hülse (122) angeordnet sind, einem ersten Satz Schlitze und einem zweiten Satz Schlitze, die sich jeweils abwärts verschiedener Bereiche der Hülse (122) erstrecken, besteht.

3. Knochenimplantat (110) nach Anspruch 1, bei dem der Knochen, für den das Implantat geeignet ist, eine Phalanx ist, die ausgewählt ist aus der Gruppe, die aus einer Grundphalanx, einer Zwischenphalanx, einer Endphalanx und einer Kombination daraus besteht.

## Revendications

1. Implant osseux (110) comprenant :
une vis (130) dotée d'une extrémité filetée (136) ; et
un manchon annulaire (122) acceptant la vis (130), le manchon (122) comprenant :
une première partie filetée (129) pour accepter l'extrémité filetée (136) de la vis (130), et
un élément d'extension (120) comprenant au moins deux fentes d'extrémités fermées (123) disposées circonférenciellement, séparant au moins deux parties dudit manchon (122) disposées sur la circonférence, lesdites au moins deux fentes (123) s'étendant le long de la longueur du manchon (122) et lesdites parties ayant une surface externe,
dans lequel l'implant (110) est implanté dans une articulation par une incision percutanée, et
dans lequel la vis (130) est pivotée autour de son axe longitudinal lorsqu'elle est accouplée à la première partie filetée (129) du manchon (122) de façon à étendre radialement les au moins deux parties disposées sur la circonférence dudit manchon (122) de sorte que lesdites surfaces externes étendent et ancrent l'implant (110) dans un os respectif,
ledit implant étant **caractérisé en ce que** ledit manchon annulaire (122) comprend en outre un crochet (237) pour ancrer l'implant dans les os respectifs.

2. Implant osseux (110) selon la revendication 1, dans lequel lesdites fentes d'extrémités fermées (123) sont choisies dans le groupe constitué par plusieurs fentes disposées en spirale le long du manchon (122), un premier ensemble et un second ensemble de fentes étendant chacun vers le bas des parties distinctes du manchon (122).

3. Implant osseux (110) selon la revendication 1, dans lequel l'os pour lequel l'implant est adapté est une phalange choisie dans le groupe constitué par une phalange proximale, une phalange intermédiaire, une phalange distale et des combinaisons de celles-ci.
